# EUROPEAN PATENT SPECIFICATION

(11) **EP 2 205 119 B1**
(45) Date of publication and mention of the grant of the patent: **21.11.2018**
(21) Application number: 08835174.7
(22) Date of filing: 02.10.2008
(51) Int. Cl.: A45D 7/02

(54) **METHOD FOR TREATING THE HAIR USING STEAM**
VERFAHREN ZUR BEHANDLUNG VON HAAR MIT DAMPF
PROCÉDÉ DE TRAITEMENT CAPILLAIRE À LA VAPEUR

(30) Priority: 03.10.2007 FR 0758025; 16.10.2007 US 960829 P
(43) Date of publication of application: 14.07.2010
(73) Proprietor: L'Oréal, 75008 Paris (FR)
(72) Inventor: RESTLE, Serge, 95390 Saint-Prix (FR); PETIT, Gaëlle, 75018 Paris (FR)
(74) Representative: Leray, Noelle
(86) International application number: PCT/EP2008/063246
(87) International publication number: WO 2009/043909

(56) References cited:
- EP-A- 0 659 397
- WO-A-2004/002262
- DE-A1- 19 810 598
- FR-A- 2 848 075
- GB-A- 2 432 310
- US-A- 3 745 306
- US-A- 4 205 692
- US-A- 4 911 185

## Description

The present invention relates to a novel method for treating hair fibres, in particular the hair.

It is known to smooth hair fibres with smoothing irons. These irons make it possible to obtain smoothing of keratinous fibres at high temperature without pulling on the hair, in contrast to blow drying. However, in order to obtain a good smooth appearance, it is necessary to carry out several passes of the iron, which considerably extends the hair treatment time. Furthermore, the repeated application of the flat smoothing irons can cause damage to the hair fibres due to the operating temperature of the smoothing iron.

It is also known to use steam for the treatment of the hair, it being possible for this steam treatment to be combined with various hair cosmetic treatments. In particular, a hair treatment method is known in the field of hair setting (nonpermanent shaping) which comprises the application of steam, for a time of less than 2 minutes at a temperature of at least 75°C, to keratinous fibres held under mechanical tension and on which a specific cosmetic composition comprising, for example, an oil, a silicone, oxidation dyes, natural dyes, direct dyes and others has been applied beforehand. Mention may be made, as examples, of the documents EP 659 395, EP 659 393, EP 659 396 and EP 659 397.

Moreover, it is known to treat the hair with smoothing irons which deliver steam. However, these irons do not make it possible to obtain a satisfactory cosmetic effect as the heat dispensed by these irons is less than that dispensed by conventional smoothing irons. Mention may in particular be made of the document WO 2004/002262, which describes a method employing such a method, the dispensed vapour additionally comprising a nonvolatile shaping cosmetic product.

These methods, although improving the cosmetic and visual properties of the hair fibres, are lengthy and often difficult to carry out. Moreover, the cosmetic effect obtained is not persistent with regard to washing.

Thus, the aim of the present invention is to develop a novel method for treating keratinous fibres which makes it possible to obtain an improvement in the cosmetic and visual properties of hair fibres with methods which are easy and rapid to carry out and which are persistent with regard to at least one washing.

This aim is achieved by the present invention, a subject-matter of which is a method for treating hair fibres as defined in claim 1.

In other words, for a treated lock of hair fibres, the stage of application of steam and the stage of shaping are not carried out simultaneously on the same portion of hair fibres, these two stages being carried out with the same device configured to carry out these two stages successively. According to the scope of the invention, the application of steam is separated from the shaping step so that when a lock is processed, the steam is applied to a portion of the lock not imprisoned into the shaping means. Besides, this process of treatment is preferably implemented with a natural drying of hair. It is understood by natural drying of hair a drying which does not include the application of blowed air or pulsed air, for example through a dry hair or a fan.

The method of the present invention makes it possible in particular to obtain rapid shaping of hair fibres while improving their cosmetic properties. Furthermore, the method of the present invention makes it possible to obtain an improvement in the cosmetic properties which is persistent with regard to time and/or at least one washing.

The hair fibres treated by the method of the invention thus exhibit an improved smoothing with a shinier appearance than that obtained with the methods of the art, this being the case even in the absence of additional cosmetic active principles.

In the context of the present invention, the term "hair fibres" is understood to mean keratinous fibres, such as the hair, or synthetic fibres, referred to as "extensions", which are intended to be attached to the hair of a person by various means, in particular by adhesive bonding, this being done in order to modify the appearance of the natural hair of a person, for example by modifying the volume, the colour or the appearance of the hair.

The application of steam is carried out before the stage of shaping the hair fibres. According to a specific embodiment, the shaping of the hair fibres is carried out after the treatment of these hair fibres with steam.

The application of steam can be repeated several times on the same fibres; however, it is possible to obtain a very good cosmetic result after a single application of steam.

According to a specific embodiment of the invention, the amount of steam is between 10 and 60 g/min.

According to a specific embodiment, this device is portable, that is to say that the tank which makes it possible to generate the steam is in contact with the part of the device comprising the orifices for dispensing the steam.

The stage of shaping the hair fibres is carried out with means of the art making it possible to obtain a temperature on the fibres of at least 50°C. The means for shaping the hair fibres are, for example, devices exhibiting a heating surface capable of coming into contact with the hair fibres and of applying a tension, even a light tension, to these fibres which makes it possible to smooth, style or disentangle the hair fibres.

According to a specific embodiment of the method of the invention, the shaping temperature is greater than 90°C, preferably between 90°C and 230°C, in particular between 90°C and 200°C, for example from 150°C to 200°C.

According to the invention, the shaping stage is carried out using smoothing irons. Mention may in particular be made of the flat smoothing irons described in Patents US 5 957 140, US 5 046 516, US 7 044 139, US 5 223 694 and US 5 091 629.

Although the shaping, in particular the smoothing, of the hair fibres can be carried out several times, results which are already very satisfactory are obtained with a single pass of the smoothing iron.

The method of the present invention makes it possible, when the application of the steam is carried out before the shaping, to satisfactorily hydrate the fibres in order to reduce the damage to the latter during the implementation of the shaping at a temperature of greater than 50°C.

The shaping stage is carried out after the treatment with steam. According to the invention, the steam application is carried out solely before the step of shaping the hair

According to a specific embodiment, the method of the invention furthermore comprises a stage of cosmetic treatment of the hair fibres using a composition comprising one or more cosmetic active principles.

This cosmetic treatment stage can be carried out before or after the application of the steam and/or before or after the shaping of the hair fibres. It should in particular be noted that this cosmetic treatment stage is not carried out simultaneously with the application of the steam.

According to a first alternative form, the method of the invention comprises, in order, the cosmetic treatment stage, followed by the application of the steam and then by the shaping of the hair fibres.

According to a second alternative form, the method of the invention comprises, in order, the application of the steam, followed by the shaping of the hair fibres and then by the cosmetic treatment stage.

According to a third alternative form, the method of the invention comprises, in order, the application of the steam, followed by the cosmetic treatment stage and then by the stage of shaping the hair fibres.

According to these two alternative forms, the method of the invention can comprise a second stage of posttreatment or pretreatment of the hair fibres, identical to or different from the first cosmetic treatment stage.

The cosmetic treatment stage can be a stage of caring for the hair fibres, which may or may not be followed by a rinsing, a stage of washing the fibres, a stage of shaping or controlling the shape, for example using a fixing gel, a shaping mousse, a lacquer or a leave-in conditioner in the cream form, a stage of permanent, semipermanent or temporary dyeing, a stage of permanent deformation using a reducing agent and optionally a fixative, or a stage of alkaline hair straightening with sodium hydroxide or with guanidine carbonate.

Mention may be made, as active cosmetic agents, of dye precursors, direct dyes, silicone or nonsilicone and fixing or nonfixing polymers, mineral, vegetable or synthetic oils, waxes, reducing agents, oxidizing agents, UV screening agents, conditioning agents, agents for combating free radicals, sequestering or stabilizing agents, antioxidants, preservatives, acidifying agents, alkaline agents, fragrances, volatile or nonvolatile silicones, reactive or chemically inert polymers, pigments, solid organic or inorganic particles, thickeners, vitamins, plant extracts, propenetrating agents or agents for swelling the fibre.

The cosmetic treatment stage can be carried out starting from a composition in the form of a thickened or nonthickened lotion, of a cream or of a gel or in any other appropriate form.

The compositions used are generally aqueous compositions which can comprise ingredients commonly used in cosmetic compositions, such as solvents, surface-active agents, thickeners, preservatives, fragrances or any other additive used in this type of composition.

According to a specific embodiment, the method of the invention can comprise an additional final stage of shaping, for example a smoothing stage, in particular if the preceding stage is a cosmetic treatment stage.

The following examples illustrate the method of the invention employed in several hair cosmetic applications.

### EXAMPLE 1

Locks of hair are washed and superficially dried. The hair is then predried until drying of approximately 80% is obtained.

Steam is applied to these predried locks under the conditions defined in the present invention, this application of steam being followed by the stage of shaping/smoothing by means of the smoothing iron heated to at least 100°C. This treatment is carried out lock by lock over the entire hair.

A permanent oxidation dye with a shade of 5.64 (coppery red light chestnut from the Majirouge range) is subsequently applied, which application consists in applying, to the hair fibres, one or more oxidation bases and one or more couplers well known in the field of permanent dyeing, in the presence of an oxidizing agent (hydrogen peroxide).

At the same time, the above test is again carried out but without employing the stage of application of steam or the stage of shaping under the preceding conditions but while carrying out smoothing with a conventional heating smoothing iron.

It is observed that the hair treated with the steam is shinier and smoother than the hair treated solely with the conventional heating smoothing iron.

After several shampooings, the colour on the hair treated with the steam is observed to last longer.

### EXAMPLE 2

A reducing cream comprising thioglycolic acid salts is applied with a brush to locks of washed and dried hair. After the setting time, the locks are again rinsed, then superficially dried and predried to 80% using a hairdryer.

Steam is applied to these predried locks under the conditions defined in the present invention, this application of steam being followed by the stage of shaping/smoothing by means of the smoothing iron heated to at least 100°C. This treatment is carried out lock by lock over the entire hair.

The fixative is subsequently applied to the entire hair. After a setting time, the locks are rinsed, superficially dried and predried.

At the same time, the above test is again carried out but without employing the stage of application of steam or the stage of shaping under the preceding conditions but while carrying out smoothing with a conventional heating smoothing iron.

It is observed that the hair treated with the method of the invention is shinier and smoother to the touch and visually. The hair is furthermore markedly better straightened with the method of the invention.

After several shampooings, it is found that the smoothing effect persists when it has been obtained with the method of the invention.

### EXAMPLE 3

The care product "Ciment thermique" from Kerastase, which is a leave-in care product comprising silicones and cationic surfactants, is applied to washed and superficially dried locks of hair. The locks are then predried until drying of approximately 80% is obtained.

Steam is applied to these predried locks under the conditions defined in the present invention, this application of steam being followed by the stage of shaping/smoothing by means of the smoothing iron heated to at least 100°C.

At the same time, the above test is again carried out but without employing the stage of application of steam or the stage of shaping under the preceding conditions but while carrying out smoothing with a conventional heating smoothing iron.

It is observed that the locks treated according to the method of the present invention are shinier and smoother to the touch and visually. The hair is markedly better smoothed.

After several shampooings, it is found that the smoothing effect persists when it has been obtained with the method of the invention.

### EXAMPLE 4

A conditioner "Nutri-Ceramide" of the Elsève trademark is applied to washed and superficially dried locks of hair. After the setting time, the locks are rinsed, then superficially dried and predried to 80% using a hairdryer.

Steam is applied to these predried locks under the conditions defined in the present invention, this application of steam being followed by the stage of shaping/smoothing by means of the smoothing iron heated to at least 100°C.

At the same time, the above test is again carried out but without employing the stage of application of steam or the stage of shaping under the preceding conditions but while carrying out smoothing with a conventional heating smoothing iron.

It is observed that the locks treated with the method of the present invention are shinier and smoother to the touch and visually. The locks are markedly better smoothed.

After several shampooings, it is found that the smoothing effect persists when it has been obtained with the method of the invention.

## Claims

1. Method for treating hair fibres which comprises:
the application to hair fibres of steam by means of a device capable of generating steam,
the shaping of these hair fibres with a smoothing iron at a temperature of greater than 50°C for smoothing the hair fibres,
these two stages are carried out with the same device configured to carry out these two stages successively, the application of steam being separated from the shaping step so that when a lock is processed, the steam is applied to a portion of the lock not imprisoned into the shaping means and the steam application is carried out solely before the step of shaping

2. Method according to Claim 1, in which the application to hair fibres of steam is carried out by means of a device capable of generating an amount of steam greater than 5 g/min.

3. Method according to Claim 1 or 2, in which the shaping temperature is greater than 90°C, preferably between 90°C and 230°C, preferably between 90 and 200°C.

4. Method according to any of claims 1 to 3, in which the smoothing is carried out in a single pass per treated lock of hair fibres.

5. Method according to any one of the preceding claims, comprising a single application of steam per lock of treated hair fibres.

6. Method according to any one of the preceding claims, additionally comprising a stage of cosmetic treatment of the hair fibres using a composition comprising one or more cosmetic active principles.

7. Method according to Claim 6, comprising, in order, the cosmetic treatment stage, followed by the stage of application of steam and then by the stage of shaping the hair fibres.

8. Method according to Claim 6, comprising, in order, the stage of application of steam, followed by the stage of shaping the hair fibres and then by the cosmetic treatment stage.

9. Method according to any one of Claims 6 to 8, in which the cosmetic treatment stage is chosen from a stage of caring for the hair fibre, a stage of washing the fibres, a stage of shaping, a stage of permanent, semipermanent or temporary dyeing, a stage of permanent deformation using a reducing agent and optionally a fixative, or a stage of alkaline straightening with sodium hydroxide or with guanidine carbonate.

## Patentansprüche

1. Verfahren zur Behandlung von Haarfasern, umfassend:
Anwenden von Dampf an Haarfasern mithilfe einer Vorrichtung, die zum Erzeugen von Dampf fähig ist,
Formen dieser Haarfasern mit einem Glätteisen bei einer Temperatur von höher als 50 °C zum Glätten der Haarfasern,
wobei diese beiden Schritte mit der gleichen Vorrichtung durchgeführt werden, die dafür gestaltet ist, diese beiden Schritte aufeinanderfolgend durchzuführen, wobei das Anwenden von Dampf von dem Schritt des Formens getrennt ist, so dass, wenn eine Locke bearbeitet wird, der Dampf an einen Teil der Locke angewendet wird, der nicht in der Formeinrichtung eingeschlossen ist, und die Dampfanwendung ausschließlich vor dem Schritt des Formens durchgeführt wird.

2. Verfahren gemäß Anspruch 1, wobei die Anwendung von Dampf an Haarfasern mithilfe einer Vorrichtung durchgeführt wird, die zum Erzeugen einer Dampfmenge von mehr als 5 g/min fähig ist.

3. Verfahren gemäß Anspruch 1 oder 2, wobei die Formtemperatur mehr als 90 °C beträgt, vorzugsweise zwischen 90 °C und 230 °C, vorzugsweise zwischen 90 und 200 °C.

4. Verfahren gemäß einem der Ansprüche 1 bis 3, wobei das Glätten in einem einzigen Durchlauf pro behandelter Locke von Haarfasern durchgeführt wird.

5. Verfahren gemäß einem der vorstehenden Ansprüche, umfassend eine einzige Anwendung von Dampf pro Locke von behandelten Haarfasern.

6. Verfahren gemäß einem der vorstehenden Ansprüche, zusätzlich umfassend einen Schritt der kosmetischen Behandlung der Haarfasern unter Verwendung einer Zusammensetzung, die ein oder mehrere kosmetische Wirkstoffe umfasst.

7. Verfahren gemäß Anspruch 6, umfassend der Reihe nach den Schritt der kosmetischen Behandlung, gefolgt von dem Schritt der Anwendung von Dampf und dann von dem Schritt des Formens der Haarfasern.

8. Verfahren gemäß Anspruch 6, umfassend der Reihe nach den Schritt der Anwendung von Dampf, gefolgt von dem Schritt des Formens der Haarfasern und dann von dem Schritt der kosmetischen Behandlung.

9. Verfahren gemäß einem der Ansprüche 6 bis 8, wobei der Schritt der kosmetischen Behandlung ausgewählt ist aus einem Schritt der Pflege der Haarfaser, einem Schritt des Waschens der Haarfasern, einem Schritt des Formens, einem Schritt des dauerhaften, halb dauerhaften oder vorübergehenden Färbens, einem Schritt des dauerhaften Verformens unter Verwendung eines Reduktionsmittels und gegebenenfalls eines Fixiermittels und einem Schritt des alkalischen Glättens mit Natriumhydroxid oder mit Guanidincarbonat.

## Revendications

1. Procédé de traitement des fibres capillaires qui comprend:
l'application sur des fibres capillaires de vapeur d'eau au moyen d'un dispositif capable de générer de la vapeur,
la mise en forme de ces fibres capillaires avec un fer à lisser à une température supérieure à 50°C pour lisser les fibres capillaires,
ces deux étapes étant réalisées avec le mème dispositive configuré pour réaliser ces deux étapes successivement, l'application de la vapeur étant dissociée de l'étape de mise en forme de sorte que lorsqu'une mèche est traitée, la vapeur est appliquée à une partie de la mèche qui n'est pas enfermée dans le moyen de mise en forme et l'application de la vapeur est uniquement effectuée avant l'étape de mise en forme.

2. Procédé selon la revendication 1 dans lequel l'application sur les fibres capillaires de vapeur est effectuée au moyen d'un dispositif capable de générer une quantité de vapeur supérieure à 5 g/min.

3. Procédé selon la revendication 1 ou 2 dans lequel la température de mise en forme est supérieure à 90 °C, de préférence entre 90 °C et 230 °C, de préférence entre 90 et 200 °C.

4. Procédé selon l'une quelconque des revendications 1 à 3 dans lequel le lissage est effectué en un seul passage par mèche de fibres capillaires traitée.

5. Procédé selon l'une quelconque des revendications précédentes comprenant une seule application de vapeur par mèche de fibres capillaires traitées.

6. Procédé selon l'une quelconque des revendications précédentes comprenant de plus une étape de traitement cosmétique des fibres capillaires au moyen d'une composition comprenant un ou plusieurs principes actifs cosmétiques.

7. Procédé selon la revendication 6 comprenant dans l'ordre l'étape de traitement cosmétique, suivie de l'étape d'application de vapeur puis de l'étape de mise en forme des fibres capillaires.

8. Procédé selon la revendication 6 comprenant dans l'ordre l'étape d'application de vapeur, suivie de l'étape de mise en forme des fibres capillaires, puis de l'étape de traitement cosmétique.

9. Procédé selon l'une quelconque des revendications 6 à 8 dans lequel l'étape de traitement cosmétique est choisie parmi une étape de soin des fibres capillaires, une étape de lavage des fibres, une étape de mise en forme, une étape de coloration permanente, semi permanente ou temporaire, une étape de déformation permanente au moyen d'un agent réducteur et éventuellement d'un agent fixateur ou une étape de défrisage alcalin avec de l'hydroxyde de sodium ou avec du carbonate de guanidine.
